# EUROPEAN PATENT APPLICATION

(11) **EP 4 831 053 A1**
(43) Date of publication of application: **28.10.2026**
(21) Application number: 24907848.6
(22) Date of filing: 27.11.2024
(51) Int. Cl.: B63B 5/24, C08K 3/04, C08K 7/06, C08K 5/00, C08K 5/132, C08K 5/3475, C08K 5/3495, C08K 5/17

(54) **ECO-FRIENDLY SHIP HULL AND METHOD FOR MANUFACTURING SAME**

(30) Priority: 20.12.2023 KR 20230187428
(71) Applicant: Ecomarine Inc., Daejeon 35229 (KR)
(72) Inventor: PARK, Deok Hun, Daejeon 34111 (KR); HONG, Moon Hyun, Daejeon 34111 (KR); MAENG, Kwang Hee, Daejeon 34111 (KR)
(74) Representative: Algemeen Octrooi- en Merkenbureau B.V.
(86) International application number: PCT/KR2024/018975
(87) International publication number: WO 2025/135563

(57) **Abstract**

An eco-friendly ship hull of the present invention is characterized by comprising: high-density polyethylene; carbon material; light stabilizer; and an antioxidant. The eco-friendly ship hull has excellent eco-friendliness, weather resistance, impact resistance, rigidity, heat resistance, and balance of these physical properties.

## Description

### [Technical Field]

The present invention relates to an eco-friendly ship hull and a method of manufacturing the same. More particularly, the present invention relates to an eco-friendly ship hull having excellent eco-friendliness, weather resistance, impact resistance, rigidity, heat resistance, and a balance of these physical properties, and a method of manufacturing the same.

### [Background Art]

Most existing small and medium-sized ships are made of fiber-reinforced plastic (FRP) materials, but FRP materials have the disadvantage of being difficult to recycle and incurring disposal costs of over 1 million won per ton. As a result, FRP ships whose service live has expired are often left in nature instead of being recycled or disposed of, which is becoming a problem. Also, FRP ships pose a problem of seriously polluting shipyard workers and the surrounding environment, as glass dust is generated during the manufacturing process, microplastics are produced when the paint used on the ships peels off, and the like.

In order to solve these problems, research was conducted on materials to replace FRP materials, and attempts were made to manufacture ships using aluminum materials. However, due to high prices and difficulties in repair, they failed to establish themselves in the market. To date, there is also no suitable alternative, so the proportion of FRP ships among Korean fishing vessels is about 97%.

Therefore, there is a need to develop eco-friendly ship hulls that are suitable for ships in terms of weather resistance, impact resistance, rigidity, and the like, are recyclable, and may reduce greenhouse gas emissions, worker health issues, and the like during the manufacturing process.

### [Disclosure]

### [Technical Problem]

Therefore, it is an object of the present invention to provide an eco-friendly ship hull having excellent eco-friendliness, weather resistance, impact resistance, rigidity, heat resistance, and a balance of these physical properties.

It is another object of the present invention to provide a method of manufacturing the eco-friendly ship hull.

The above and other objects of the present invention can all be achieved by the present invention described below.

### [Technical Solution]

1. One aspect of the present invention is directed to providing an eco-friendly ship hull. The eco-friendly ship hull includes high-density polyethylene; a carbon material; a light stabilizer; and an antioxidant.
2. In embodiment 1, the eco-friendly ship hull may include about 100 parts by weight of the high-density polyethylene; about 0.01 to about 4 parts by weight of the carbon material; about 0.01 to about 2 parts by weight of the light stabilizer; and about 0.01 to about 2 parts by weight of the antioxidant.
3. In embodiment 1 or 1, the high-density polyethylene may be linear high-density polyethylene.
4. In any one of embodiments 1 to 3, the high-density polyethylene may have a weight average molecular weight of about 100,000 to about 1,000,000 g/mol.
5. In any one of embodiments 1 to 4, the carbon material may include one or more of carbon black, carbon fibers, carbon nanotubes, graphite, and graphene.
6. In any one of embodiments 1 to 5, the light stabilizer may include one or more of an ultraviolet (UV) absorber, a hindered amine-based light stabilizer, and a UV stabilizer.
7. In any one of embodiments 1 to 6, the antioxidant may include one or more of a hindered phenol compound, an aromatic amine compound, a phosphite compound, a phosphonite compound, an organic sulfur-containing compound, and a dithiophosphonate compound.
8. In any one of embodiments 1 to 7, the eco-friendly ship hull may further include an additive including one or more of a flame retardant, an anti-dripping agent, an anti-bacterial agent, a heat stabilizer, a lubricant, a release agent, a nucleating agent, an anti-static agent, a dispersant, a processing aid, a compatibilizer, and a pigment.
9. In any one of embodiments 1 to 8, the pigment may include one or more of a monoazo pigment, a diazo pigment, an anthraquinone pigment, a benzimidazole pigment, a quinacridone pigment, a quinophthalone pigment, a diketopyrrolopyrrole pigment, a dioxazine pigment, an indanthrone pigment, an isoindoline pigment, an isoindolinone pigment, a metal complex pigment, a perinone pigment, a perylene pigment, a phthalocyanine pigment, and a non-neutral pigment.
10. In any one of embodiments 1 to 9, the eco-friendly ship hull may have a tensile strength of about 24 N/mm² or more for a 3.2 mm-thick specimen, as measured according to ASTM D638.
11. In any one of embodiments 1 to 10, the eco-friendly ship hull may have a notched Izod impact strength of about 196 J/m or more for a 3.2 mm-thick specimen, as measured according to ASTM D256.
12. In any one of embodiments 1 to 11, the eco-friendly ship hull may not undergo brittle fracture when a 3.2 mm-thick specimen is irradiated with UV rays at 340 nm and 0.35 W/m² conditions at 65°C for 5,000 hours according to ASTM G155 and the notched Izod impact strength is then measured at -20°C according to ASTM D256.
13. In any one of embodiments 1 to 12, the eco-friendly ship hull may have a difference in yellowness index (ΔYI₁₋₀) of about 2.5 or less before and after UV irradiation, as measured according to the following Equation 1, and may have a difference in yellowness index (ΔYI₁₋₀) of about 2.5 or less before and after UV irradiation, as measured according to the following Equation 2:
   [Equation 1] 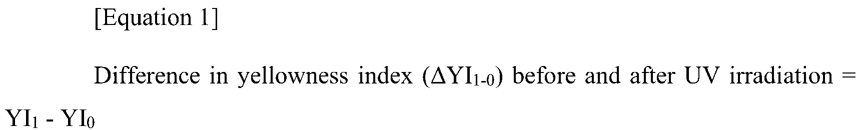 Difference in yellowness index (ΔYI1-0) before and after UV irradiation = YI1 - YI0
   wherein YI₀ is a yellowness index of a 3.2 mm-thick specimen before UV irradiation, as measured according to ASTM D1925, and YI₁ is a yellowness index of the specimen after UV irradiation, as measured according to ASTM D1925 after irradiating the specimen with UV rays at 340 nm and 0.35 W/m² conditions at 65°C for 12,000 hours according to ASTM G155; [Equation 2] 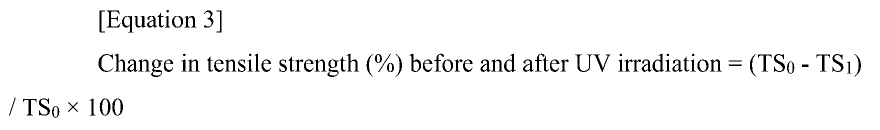 Difference in yellowness index (ΔYI2-0) before and after UV irradiation = YI2 - YI0
   wherein YI₀ is a yellowness index of a 3.2 mm-thick specimen before UV irradiation, as measured according to ASTM D1925, and YI₂ is a predicted yellowness index value of the specimen after UV irradiation, as measured according to ASTM D1925 after irradiating the specimen with UV rays at 340 nm and 0.35 W/m² conditions at 65°C for 16,000 hours according to ASTM G155.
14. In any one of embodiments 1 to 13, the eco-friendly ship hull may have a change in tensile strength of about 12% or less before and after UV irradiation, as measured according to the following Equation 3, and may have a change in tensile strength of about 12% or less before and after UV irradiation, as measured according to the following Equation 4: [Equation 3] 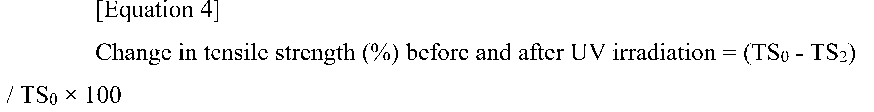 Change in tensile strength (%) before and after UV irradiation = (TS0 - TS1) / TS0 × 100
   wherein TS₀ is a tensile strength of a 3.2 mm-thick specimen before UV irradiation, as measured according to ASTM D638, and TS₁ is a tensile strength of the specimen after UV irradiation, as measured according to ASTM D638 after irradiating the specimen with UV rays at 340 nm and 0.35 W/m² conditions at 65°C for 12,000 hours according to ASTM G155; [Equation 4] 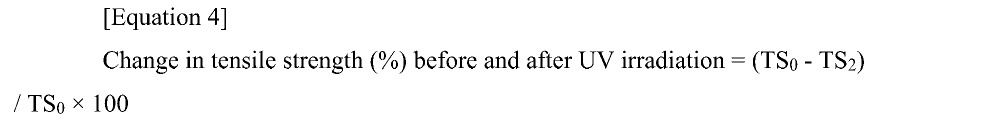 Change in tensile strength (%) before and after UV irradiation = (TS0 - TS2) / TS0 × 100
   wherein TS₀ is a tensile strength of a 3.2 mm-thick specimen before UV irradiation, as measured according to ASTM D638, and TS₂ is a predicted tensile strength value of the specimen after UV irradiation, as measured according to ASTM D638 after irradiating the specimen with UV rays at 340 nm and 0.35 W/m² conditions at 65°C for 16,000 hours according to ASTM G155.
15. In any one of embodiments 1 to 14, the eco-friendly ship hull may have an anti-slip surface shape having a cubic or grain shape.
16. In any one of embodiments 1 to 15, the eco-friendly ship hull may have a glossy surface or a matte surface.
17. Another aspect of the present invention is directed to providing a method of manufacturing an eco-friendly ship hull. The manufacturing method is characterized by including: mixing and melting high-density polyethylene, a carbon material, a light stabilizer, and an antioxidant and then extruding the resulting mixture to prepare pellets; extrusion-molding the pellets into a sheet shape; cutting the sheet into the shape of a ship hull part according to a nesting drawing; and assembling (welding) the ship hull part.
18. In embodiment 17, the pellets may be prepared under extrusion molding temperature conditions of about 170 to about 240°C.
19. In embodiment 17 or 18, the sheet may have a thickness of about 5 mm or more and a width of about 500 mm or more.
20. In any one of embodiments 17 to 19, the sheet may have a single-layered structure or a multi-layered structure.

### [Advantageous Effects]

The present invention has the inventive effect of providing an eco-friendly ship hull having excellent eco-friendliness, weather resistance, impact resistance, rigidity, heat resistance, and a balance of these physical properties, and a method of manufacturing the same.

### [Description of Drawings]

FIG. 1 is a nesting drawing for manufacturing an eco-friendly ship hull according to one exemplary embodiment of the present invention.
FIG. 2 is a cross-sectional view of an eco-friendly ship hull sheet having a single-layered structure according to one exemplary embodiment of the present invention.
FIG. 3 is a cross-sectional view of an eco-friendly ship hull sheet having a multi-layered structure according to one exemplary embodiment of the present invention.
FIG. 4 is a perspective view of an eco-friendly ship hull sheet having an anti-slip surface formed thereon according to one exemplary embodiment of the present invention.
FIG. 5 is a cross-sectional view of the eco-friendly ship hull sheet having an anti-slip surface formed thereon according to one exemplary embodiment of the present invention.

### [Best Mode]

Hereinafter, the present invention will be described in detail, as follows.

An eco-friendly ship hull according to the present invention includes (A) high-density polyethylene; (B) a carbon material; (C) a light stabilizer; and (D) an antioxidant.

As used herein to represent a specific numerical range, the expression "a to b" means "≥ a and ≤ b".

### (A) High-density polyethylene

High-density polyethylene (HDPE) according to one exemplary embodiment of the present invention serves to replace a fiber-reinforced plastic (FRP) material and aluminum used in existing ships, thereby imparting eco-friendliness (recyclability, greenhouse gas reduction, and the like) to the products. HDPE may be applied along with a carbon material, a light stabilizer, an antioxidant, and the like to improve the eco-friendliness, weather resistance, impact resistance, rigidity, heat resistance, and the balance of these physical properties of the eco-friendly ship hull.

In an exemplary embodiment, the high-density polyethylene may be linear high-density polyethylene.

In an exemplary embodiment, the high-density polyethylene may have a weight average molecular weight (Mw) of about 100,000 to about 1,000,000 g/mol, for example, about 120,000 to about 980,000 g/mol, and a density of about 0.94 to about 0.97 g/cm³, as measured by gel permeation chromatography (GPC) at 160°C under 1,2,4-trichlorobenzene (TCB) conditions. Within the above ranges, the eco-friendly ship hull may have excellent rigidity, impact resistance, and the like.

### (B) Carbon material

The carbon material according to one exemplary embodiment of the present invention may be applied to high-density polyethylene along with a light stabilizer, an antioxidant, and the like to improve the eco-friendliness, weather resistance, impact resistance, rigidity, heat resistance, and the balance of these physical properties of the eco-friendly ship hull.

In an exemplary embodiment, the carbon material may include carbon black, carbon fibers, carbon nanotubes, graphite, graphene, and a combination thereof.

In an exemplary embodiment, the carbon material may have an average particle size of about 5 to about 50 nm, for example, about 10 to about 45 nm, as measured by transmission electron microscopy (TEM). Within the above range, the weather resistance (long-term property maintenance) of the eco-friendly ship hull may be excellent due to the increased surface area and the increased UV absorption rate.

In an exemplary embodiment, the carbon material may be included in an amount of about 0.01 to about 4 parts by weight, for example, about 1 to about 3 parts by weight, based on about 100 parts by weight of the high-density polyethylene. Within the above range, the weather resistance (UV degradation prevention) of the eco-friendly ship hull may be excellent.

### (C) Light stabilizer

The light stabilizer according to one exemplary embodiment of the present invention may be applied to high-density polyethylene along with a carbon material, an antioxidant, and the like to improve the eco-friendliness, durability, impact resistance, rigidity, heat resistance, and the balance of these physical properties of the eco-friendly ship hull. In this case, commercially available light stabilizers used in conventional thermoplastic resin compositions may be used.

In an exemplary embodiment, the light stabilizer may include one or more of a UV absorber, a hindered amine-based light stabilizer, and a UV stabilizer.

In an exemplary embodiment, the UV absorber may include a benzophenone-based UV absorber, a benzotriazole-based UV absorber, a salicylic acid-based UV absorber, a cyanoacrylate-based UV absorber, and a combination thereof.

Examples of the benzophenone-based UV absorber may include 2,4-dihydroxybenzophenone, 2-hydroxy-4-methoxybenzophenone, 2-hydroxy-4-octoxybenzophenone, 2-hydroxy-4-dodecyloxy benzophenone, 2,2'-dihydroxy-4-dimethoxybenzophenone, 2,2'-dihydroxy-4,4'-dimethoxybenzophenone, 2-hydroxy-4-methoxy-5-sulfobenzophenone, bis(2-methoxy-4-hydroxy-5-benzoyl phenyl)methane, and a combination thereof, but the present invention is not limited thereto.

Examples of the benzotriazole-based UV absorber may include 2-(2'-hydroxy-5'-methylphenyl)benzotriazole, 2-(2'-hydroxy-5'-tert-butylphenyl)benzotriazole, 2-(2'-hydroxy-3',5'-di-tert-butylphenyl)benzotriazole, 2-(2'-hydroxy-3'-tert-butyl-5'-methylphenyl)-5-chlorobenzotriazole, 2-(2'-hydroxy-3',5'-di-tert-butylphenyl)5-chlorobenzotriazole,2-(2'-hydroxy-3',5'-di-tert-amyl phenyl)benzotriazole, 2-(2'-hydroxy-4'-octoxyphenyl)benzotriazole, 2-[2'-hydroxy-3'-(3",4",5",6",-tetrahydrophthalimidemethyl)-5'-methylphenyl]benzotriazole, 2,2'methylenebis[4-(1,1,3,3-tetramethylbutyl)-6-(2H-benzotriazole-2-yl)phenol], [2(2'-hydroxy-5'-methacryloxyphenyl)-2H-benzotriazole, and a combination thereof, but the present invention is not limited thereto.

Examples of the salicylic acid-based UV absorber may include phenylsalicylate, p-tert-butylphenylsalicylate, p-octylphenylsalicylate, and a combination thereof, but the present invention is not limited thereto.

Examples of the cyanoacrylate-based UV absorber may include 2-ethylhexyl-2-cyano-3,3'-diphenylacrylate, ethyl-2-cyano-3,3'-diphenylacrylate, and a combination thereof, but the present invention is not limited thereto.

Examples of the hindered amine-based light stabilizer may include [bis(2,2,6,6-tetramethyl-4-piperidyl)sebacate], bis(1,2,2,6,6-pentamethyl-4-piperidyl)sebacate, methyl1,2,2,6,6-pentamethyl-4-piperidylsebacate, and a combination thereof, but the present invention is not limited thereto.

Examples of the UV stabilizer may include nickel bis(octylphenyl)sulfide, [2,2'-thiobis(4-tert-octylphenolate)]-n-butylaminenickel, nickel complex-3,5-di-tert-butyl-4-hydroxybenzyl-phosphate monoethylate, nickel-dibutyldithiocarbamate, a benzoate-type quencher, nickel-dibutyldithiocarbamate, and a combination thereof, but the present invention is not limited thereto.

In an exemplary embodiment, the light stabilizer may be included in an amount of about 0.01 to about 2 parts by weight, for example, about 0.05 to about 1 part by weight, based on about 100 parts by weight of the high-density polyethylene. Within the above range, the weather resistance (yellowness index maintenance, and the like) of the eco-friendly ship hull may be excellent.

### (D) Antioxidant

The antioxidant according to one exemplary embodiment of the present invention may be applied to high-density polyethylene along with a carbon material, a light stabilizer, and the like to improve the eco-friendliness, durability, impact resistance, rigidity, heat resistance, and the balance of these physical properties of the eco-friendly ship hull. In this case, antioxidants used in conventional thermoplastic resin compositions may be used.

In an exemplary embodiment, the antioxidant may include a hindered phenol compound, an aromatic amine compound, a phosphite compound, a phosphonite compound, an organic sulfur-containing compound, a dithiophosphonate compound, and a mixture thereof.

In an exemplary embodiment, commercially available products may be used as the antioxidant. In this case, Irganox 1010, Irgafos 168, or a mixture thereof may be used, but the present invention is not limited thereto.

In an exemplary embodiment, the antioxidant may be included in an amount of about 0.01 to about 2 parts by weight, for example, about 0.05 to about 1 part by weight, based on about 100 parts by weight of the high-density polyethylene. Within the above range, an oxidation heat reaction during the manufacture of pellets and sheets for manufacturing an eco-friendly ship hull may be suppressed, and processing stability and the like may be excellent.

The eco-friendly ship hull according to one exemplary embodiment of the present invention may further include an additive included in a conventional thermoplastic resin composition. Examples of the additive may include a flame retardant, an anti-dripping agent, an anti-bacterial agent, a heat stabilizer, a lubricant, a release agent, a nucleating agent, an anti-static agent, a dispersant, a processing aid, a compatibilizer, a pigment, and a mixture thereof, but the present invention is not limited thereto.

In an exemplary embodiment, the pigment may be used as a pigment alone or in the form of a pigment masterbatch (MB).

In an exemplary embodiment, the pigment may include a monoazo pigment, a diazo pigment, an anthraquinone pigment, a benzimidazole pigment, a quinacridone pigment, a quinophthalone pigment, a diketopyrrolopyrrole pigment, a dioxazine pigment, an indanthrone pigment, an isoindoline pigment, an isoindolinone pigment, a metal complex pigment, a perinone pigment, a perylene pigment, a phthalocyanine pigment, a non-neutral pigment, and a mixture thereof.

In an exemplary embodiment, when the pigment is applied, various colors may be imparted to the ship without the environmental pollution issues associated with the use of paint in conventional ship manufacturing.

In an exemplary embodiment, when the additive is used, the content of the additive may be about 0.001 to about 40 parts by weight, for example, about 0.01 to about 10 parts by weight, based on about 100 parts by weight of the high-density polyethylene, but the present invention is not limited thereto.

The eco-friendly ship hull according to one exemplary embodiment of the present invention may be manufactured by a manufacturing method, which includes: mixing and melting the components and then extruding the resulting mixture to prepare pellets; extrusion-molding the pellets into a sheet shape; cutting the sheet into the shape of a ship hull part according to a nesting drawing; and assembling (welding) the ship hull part.

In an exemplary embodiment, the pellets may be prepared using a conventional twin-screw extruder under extrusion molding temperature conditions of about 170 to about 240°C, for example, about 190 to about 230°C.

In an exemplary embodiment, the sheet may be manufactured using a multi-layered twin-screw extruder under extrusion molding temperature conditions of about 170 to about 240°C, for example, about 190 to about 230°C. Such a sheet extrusion molding method is well known to those skilled in the art to which the present invention pertains.

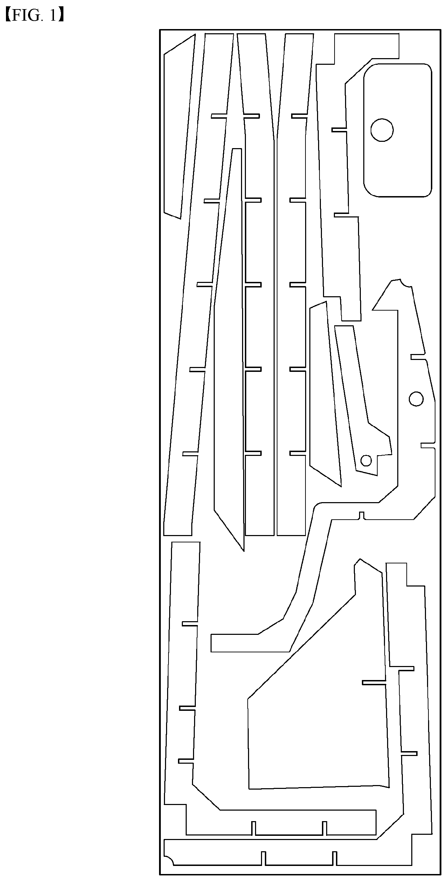

FIG. 1 is a nesting drawing for manufacturing an eco-friendly ship hull according to one exemplary embodiment of the present invention. The eco-friendly ship hull may be manufactured by cutting the sheet into the shape of a ship hull part according to a design (e.g., the nesting drawing shown in FIG. 1) and then assembling (welding) the ship hull part. Such cutting and assembly (welding) methods are well known to those skilled in the art to which the present invention pertains. For example, examples of the cutting method may include a CNC method, a water-jet method, and the like, but the present invention is not limited thereto.

In an exemplary embodiment, the sheet may have a thickness of about 5 mm or more, for example, about 5 to about 100 mm, and a width of about 500 mm or more, for example, about 500 to about 2,000 mm. Within the above range, depending on the application location of the hull, the design and manufacture of the eco-friendly ship hull may be facilitated.

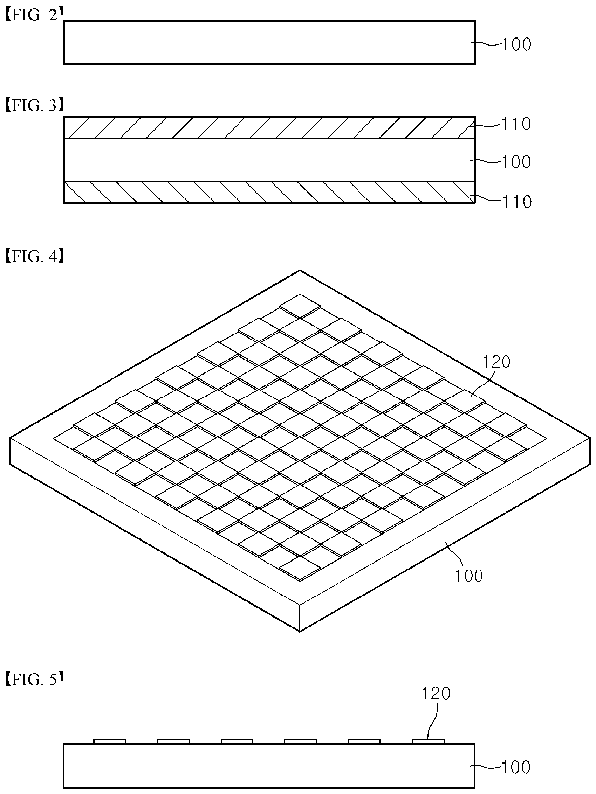

FIG. 2 is a cross-sectional view of an eco-friendly ship hull sheet having a single-layered structure according to one exemplary embodiment of the present invention, and FIG. 3 is a cross-sectional view of an eco-friendly ship hull sheet having a multi-layered structure according to one exemplary embodiment of the present invention. As shown in FIGS. 2 and 3, the sheet may have a single-layered structure or a multi-layered structure.

In an exemplary embodiment, the sheet having the multi-layered structure may have a structure in which a laminated sheet 110, for example, a laminated film configured to ensure flame retardancy, anti-fouling function, hull design, and the like, or a sheet of 2 layers or more, is laminated on at least one surface of a sheet 100 having a single-layered structure.

FIG. 4 is a perspective view of an eco-friendly ship hull sheet having an anti-slip surface formed thereon according to one exemplary embodiment of the present invention, and FIG. 5 is a cross-sectional view of the eco-friendly ship hull sheet having an anti-slip surface formed thereon according to one exemplary embodiment of the present invention. As shown in FIGS. 4 and 5, the eco-friendly ship hull (sheet) may include an anti-slip surface 120 having a cubic or grain shape on at least one surface of the sheet 100. Also, when the sheet has a multi-layered structure, the anti-slip surface 120 may also be formed on the laminated sheet 110.

In an exemplary embodiment, the eco-friendly ship hull (sheet) may have a glossy surface or a matte surface, when necessary.

In an exemplary embodiment, the eco-friendly ship hull may have a tensile strength of about 24 N/mm² or more for a 3.2 mm-thick specimen, as measured according to ASTM D638.

In an exemplary embodiment, the eco-friendly ship hull may have a notched Izod impact strength of about 196 J/m or more for a 3.2 mm-thick specimen, as measured at -20°C according to ASTM D256.

In an exemplary embodiment, the eco-friendly ship hull may not undergo brittle fracture when a 3.2 mm-thick specimen is irradiated with UV rays at 340 nm and 0.35 W/m2 conditions at 65°C for 5,000 hours according to ASTM G155 and the notched Izod impact strength is then measured at -20°C according to ASTM D256.

In an exemplary embodiment, the eco-friendly ship hull may have a difference in yellowness index (ΔYI1-0) of about 2.5 or less, for example, about 1 or less, before and after UV irradiation, as measured according to the following Equation 1 or 2. [Equation 1] 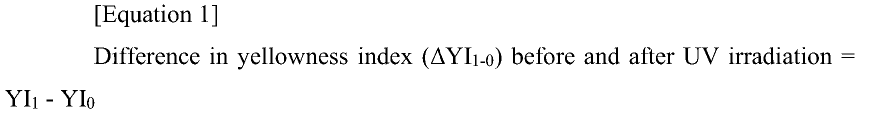 Difference in yellowness index (ΔYI1-0) before and after UV irradiation = YI1 - YI0
wherein YI₀ is a yellowness index of a 3.2 mm-thick specimen before UV irradiation, as measured according to ASTM D1925, and YI₁ is a yellowness index of the specimen after UV irradiation, as measured according to ASTM D1925 after irradiating the specimen with UV rays at 340 nm and 0.35 W/m² conditions at 65°C for 12,000 hours according to ASTM G155. [Equation 2] 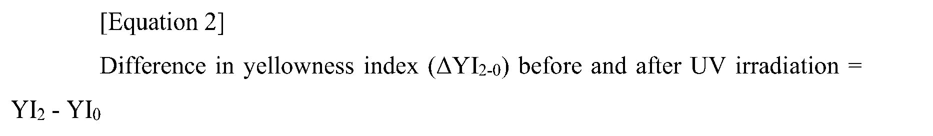 Difference in yellowness index (ΔYI2-0) before and after UV irradiation = YI2 - YI0
wherein YI₀ is a yellowness index of a 3.2 mm-thick specimen before UV irradiation, as measured according to ASTM D1925, and YI₂ is a predicted yellowness index value of the specimen after UV irradiation, as measured according to ASTM D1925 after irradiating the specimen with UV rays at 340 nm and 0.35 W/m² conditions at 65°C for 16,000 hours according to ASTM G155.

In an exemplary embodiment, the eco-friendly ship hull may have a change in tensile strength of about 12% or less, for example, about 10% or less, before and after UV irradiation, as measured according to the following Equation 3 or 4. [Equation 3] 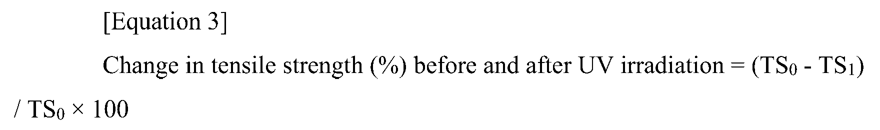 Change in tensile strength (%) before and after UV irradiation = (TS0 - TS1) / TS0 × 100
wherein TS₀ is a tensile strength of a 3.2 mm-thick specimen before UV irradiation, as measured according to ASTM D638, and TS₁ is a tensile strength of the specimen after UV irradiation, as measured according to ASTM D638 after irradiating the specimen with UV rays at 340 nm and 0.35 W/m² conditions at 65°C for 12,000 hours according to ASTM G155. [Equation 4] 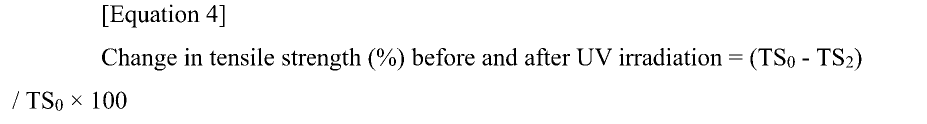 Change in tensile strength (%) before and after UV irradiation = (TS0 - TS2) / TS0 × 100
wherein TS₀ is a tensile strength of a 3.2 mm-thick specimen before UV irradiation, as measured according to ASTM D638, and TS₂ is a predicted tensile strength value of the specimen after UV irradiation, as measured according to ASTM D638 after irradiating the specimen with UV rays at 340 nm and 0.35 W/m² conditions at 65°C for 16,000 hours according to ASTM G155.

The eco-friendly ship hull according to the present invention is suitable for use in ships in terms of weather resistance, impact resistance, rigidity, and the like. Unlike conventional FRP ships, it is recyclable after use, and is eco-friendly as it may reduce greenhouse gas emissions, worker health issues, and the like during the ship manufacturing process. Also, the eco-friendly ship hull is economical compared to aluminum ships, which require high manufacturing and repair costs.

### [Mode for Invention]

Hereinafter, the present invention will be described in more detail with reference to the following examples. However, it should be understood that these examples are provided for illustrative purposes only and are not interpreted as limiting the present invention.

### EXAMPLE

Hereinafter, the specifications of each component used in the examples and comparative examples are as follows.

### (A) High-density polyethylene

High-density polyethylene (HDPE, Manufacturer: Lotte Chemical, Product Name: 5200B, Weight average molecular weight: about 250,000 g/mol) was used.

### (B) Carbon material

Carbon black (Manufacturer: Burra Carbon, Product Name: Raven UV Ultra) was used.

### (C) Light stabilizer

A light stabilizer (1,6-hexanediamine, N,N'-bis(2,2,6,6-tetramethyl-4,4-piperidinyl)-, polymers with 2,4-dichloro-6-(4-morpholinyl)-1,3,5-triazine, Manufacturer: Syensqo, Product Name: UV3346) was used.

### (D) Antioxidant

Pentaerythritol tetrakis [3-(3,5-di-t-butyl-4-hydroxyphenyl)-propionate] (Manufacturer: Ciba Specialty Chemicals, Product Name: Irganox 1010) and tris(2,4-di-t-butylphenyl)phosphite (Manufacturer: Ciba Specialty Chemicals, Product Name: Irgafos 168) were mixed (mixing ratio (Irganox 101:Irgafos 1680) = 1:1.5) and used.

### Example 1 and Comparative Examples 1 to 3

The respective components were added in the amounts listed in Table 1 below, and then extruded at about 220°C to prepare pellets. A twin-screw extruder with L/D = 36 and a diameter of 45 mm was used for extrusion. The prepared pellets were dried at about 80°C for about 2 hours, and then injection-molded in a 220-ton injection molding machine (molding temperature: about 220°C, die temperature: about 40°C) to manufacture a specimen. The physical properties of the manufactured specimen were evaluated by the following methods. The results are shown in Table 1 below.

### Property evaluation

(1) Density (Units: g/cm³): The density of the sheet was measured by the submersed displacement method (after 24 hours) according to ISO 1183.
(2) Melt flow Index (Units: g/10 min): The melt flow index (MI) was measured under conditions of 190°C and 2.16 kgf according to ASTM D1238.
(3) Tensile strength (Units: N/mm²): The tensile strength of a 3.2 mm-thick specimen was measured according to ASTM D638.
(4) Flexural strength (Units: N/mm²): The flexural strength of a 3.2 mm-thick specimen was measured according to ASTM D790.
(5) Flexural modulus (Units: N/mm²): The flexural modulus of a 3.2 mm-thick specimen was measured according to ASTM D790.
(6) Notched Izod impact strength (Units: J/m): The notched Izod impact strength of a 3.2 mm-thick specimen was measured at -20°C according to ASTM D256.
(7) Notched Izod impact strength (Units: J/m) after UV irradiation: Weather resistance was evaluated by irradiating a 3.2 mm-thick specimen with UV rays at 340 nm and 0.35 W/m² at 65°C for 5,000 hours according to ASTM G155 and then measuring the notched Izod impact strength at -20°C according to ASTM D256. (NB: Non-Break)
(8) Heat deformation temperature (Units: °C): The heat deformation temperature was measured according to ASTM D640 when a deformation of 0.254 mm occurred on the surface of the specimen.
(9) Vicat softening point (Units: °C): The Vicat softening point was measured according to ISO 306 when needle-like particles penetrated 1 mm into the surface of the specimen.
(10) Weather resistance evaluation: The difference in yellowness index (ΔYI₁₋₀) before and after UV irradiation was measured according to the following Equations 1 and 2. [Equation 1] 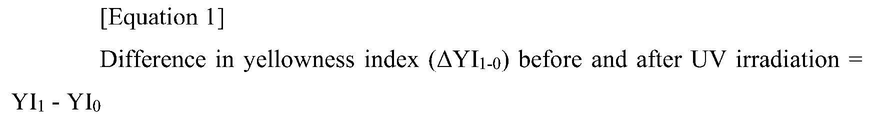 Difference in yellowness index (ΔYI1-0) before and after UV irradiation = YI1 - YI0
   wherein YI₀ is a yellowness index of a 3.2 mm-thick specimen before UV irradiation, as measured according to ASTM D1925, and YI₁ is a yellowness index of the specimen after UV irradiation, as measured according to ASTM D1925 after irradiating the specimen with UV rays at 340 nm and 0.35 W/m² conditions at 65°C for 12,000 hours according to ASTM G155. [Equation 2] 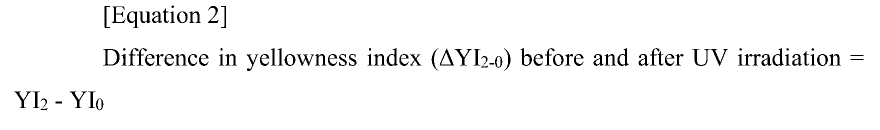 Difference in yellowness index (ΔYI2-0) before and after UV irradiation = YI2 - YI0
   wherein YI₀ is a yellowness index of a 3.2 mm-thick specimen before UV irradiation, as measured according to ASTM D1925, and YI₂ is a predicted yellowness index value of the specimen after UV irradiation, as measured according to ASTM D1925 after irradiating the specimen with UV rays at 340 nm and 0.35 W/m² conditions at 65°C for 16,000 hours according to ASTM G155.
(11) Weather resistance evaluation: The change in tensile strength (Units: %) before and after UV irradiation was measured according to the following Equations 3 and 4. [Equation 4] 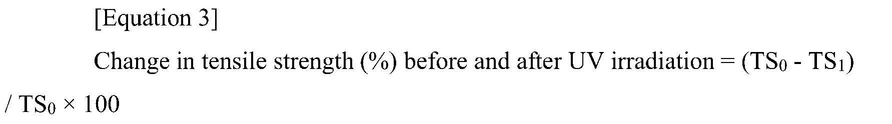 Change in tensile strength (%) before and after UV irradiation = (TS0 - TS1) / TS0 × 100
   wherein TS₀ is a tensile strength of a 3.2 mm-thick specimen before UV irradiation, as measured according to ASTM D638, and TS₁ is a tensile strength of the specimen after UV irradiation, as measured according to ASTM D638 after irradiating the specimen with UV rays at 340 nm and 0.35 W/m² conditions at 65°C for 12,000 hours according to ASTM G155. [Equation 4] 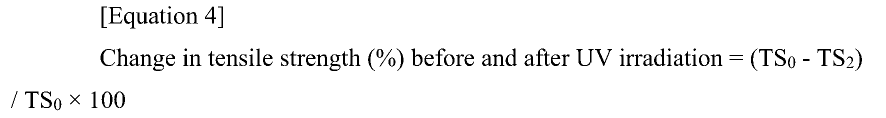 Change in tensile strength (%) before and after UV irradiation = (TS0 - TS2) / TS0 × 100
   wherein TS₀ is a tensile strength of a 3.2 mm-thick specimen before UV irradiation, as measured according to ASTM D638, and TS₂ is a predicted tensile strength value of the specimen after UV irradiation, as measured according to ASTM D638 after irradiating the specimen with UV rays at 340 nm and 0.35 W/m² conditions at 65°C for 16,000 hours according to ASTM G155.

**Table 1**

| | Example | Comparative Examples | | |
|---|---|---|---|---|
| | 1 | 1 | 2 | 3 |
| (A) (parts by weight) | 100 | 100 | 100 | 100 |
| (B) (parts by weight) | 2.5 | - | 2.5 | 2.5 |
| (C) (parts by weight) | 0.2 | 0.1 | - | 0.1 |
| (D) (parts by weight) | 0.25 | 0.25 | 0.25 | - |
| Density | 0.969 | 0.96 | 0.969 | 0.964 |
| Melt index | 0.25 | 0.36 | 0.25 | 0.27 |
| Tensile strength | 29 | 26 | 29 | 27 |
| Flexural strength | 29 | 23 | 28 | 26 |
| Flexural modulus | 1,381 | 1,350 | 1,395 | 1,350 |
| Notched Izod impact strength | 373 | 343 | 353 | 343 |
| Notched Izod impact strength after UV irradiation | ≥ 373 (NB) | - | 33.3 | - |
| Heat deformation temperature | 99 | 94 | 97 | 95 |
| Vicat softening point | 127 | 125 | 126 | 127 |
| Difference in yellowness index before and after UV irradiation (before and after 12,000 hours of irradiation) | 1.68 | - | 3.77 | - |
| Difference in yellowness index before and after UV irradiation (before and after 16,000 hours of irradiation) | about 2.5 | - | about 5 | - |
| Change in tensile strength before and after UV irradiation (before and after 12,000 hours of irradiation) | about 8 | - | about 20 | - |
| Change in tensile strength before and after UV irradiation (before and after 16,000 hours of irradiation) | about 10 | - | about 30 | - |

Based on the above results, it can be seen that the eco-friendly ship hull of the present invention is eco-friendly because high-density polyethylene is applied instead of an FRP material, and exhibits excellent weather resistance (notched Izod impact strength after UV irradiation, difference in yellowness index before and after UV irradiation, change in tensile strength before and after UV irradiation), impact resistance (notched Izod impact strength), rigidity (tensile strength, flexural strength, flexural modulus), heat resistance (heat deformation temperature, Vicat softening point), and a balance of these physical properties.

On the other hand, in the case of Comparative Example 1, in which a carbon material was not applied, it can be seen that weather resistance and the like deteriorated because the physical properties were not measurable after UV irradiation (specimen destruction). In the case of Comparative Example 2, in which a light stabilizer was not applied, it can be seen that weather resistance and the like deteriorated and impact resistance and the like was reduced compared to those of Example 1. In the case of Comparative Example 3, in which an antioxidant was not applied, it can be seen that weather resistance and the like deteriorated and processability and the like may be reduced because the physical properties were not measurable after UV irradiation (specimen destruction).

Although some embodiments have been described herein, it will be understood by those skilled in the art that various modifications, changes, and alterations can be made without departing from the spirit and scope of the invention. Therefore, it should be understood that these embodiments are provided for illustration only and are not to be construed in any way as limiting the present invention. The scope of the present invention should be defined by the appended claims rather than by the foregoing description, and the claims and equivalents thereto are intended to cover such modifications and the like as would fall within the scope of the present invention.

## Claims

1. An eco-friendly ship hull comprising:
high-density polyethylene;
a carbon material;
a light stabilizer; and
an antioxidant.

2. The eco-friendly ship hull of claim 1, wherein the eco-friendly ship hull comprises about 100 parts by weight of the high-density polyethylene; about 0.01 to about 4 parts by weight of the carbon material; about 0.01 to about 2 parts by weight of the light stabilizer; and about 0.01 to about 2 parts by weight of the antioxidant.

3. The eco-friendly ship hull of claim 1 or 2, wherein the high-density polyethylene is linear high-density polyethylene.

4. The eco-friendly ship hull of any one of claims 1 to 3, wherein the high-density polyethylene has a weight average molecular weight of about 100,000 to about 1,000,000 g/mol.

5. The eco-friendly ship hull of any one of claims 1 to 4, wherein the carbon material includes one or more of carbon black, carbon fibers, carbon nanotubes, graphite, and graphene.

6. The eco-friendly ship hull of any one of claims 1 to 5, wherein the light stabilizer includes one or more of an ultraviolet (UV) absorber, a hindered amine-based light stabilizer, and a UV stabilizer.

7. The eco-friendly ship hull of any one of claims 1 to 6, wherein the antioxidant includes one or more of a hindered phenol compound, an aromatic amine compound, a phosphite compound, a phosphonite compound, an organic sulfur-containing compound, and a dithiophosphonate compound.

8. The eco-friendly ship hull of any one of claims 1 to 7, wherein the eco-friendly ship hull further includes an additive including one or more of a flame retardant, an anti-dripping agent, an anti-bacterial agent, a heat stabilizer, a lubricant, a release agent, a nucleating agent, an anti-static agent, a dispersant, a processing aid, a compatibilizer, and a pigment.

9. The eco-friendly ship hull of claim 8, wherein the pigment includes one or more of a monoazo pigment, a diazo pigment, an anthraquinone pigment, a benzimidazole pigment, a quinacridone pigment, a quinophthalone pigment, a diketopyrrolopyrrole pigment, a dioxazine pigment, an indanthrone pigment, an isoindoline pigment, an isoindolinone pigment, a metal complex pigment, a perinone pigment, a perylene pigment, a phthalocyanine pigment, and a non-neutral pigment.

10. The eco-friendly ship hull of any one of claims 1 to 9, wherein the eco-friendly ship hull has a tensile strength of about 24 N/mm² or more for a 3.2 mm-thick specimen, as measured according to ASTM D638.

11. The eco-friendly ship hull of any one of claims 1 to 10, wherein the eco-friendly ship hull has a notched Izod impact strength of about 196 J/m or more for a 3.2 mm-thick specimen, as measured at -20°C according to ASTM D256.

12. The eco-friendly ship hull of any one of claims 1 to 11, wherein the eco-friendly ship hull does not undergo brittle fracture when a 3.2 mm-thick specimen is irradiated with ultraviolet (UV) rays at 340 nm and 0.35 W/m² conditions at 65°C for 5,000 hours according to ASTM G155 and the notched Izod impact strength is then measured at -20°C according to ASTM D256.

13. The eco-friendly ship hull of any one of claims 1 to 12, wherein the eco-friendly ship hull has a difference in yellowness index (ΔYI₁₋₀) of about 2.5 or less before and after ultraviolet (UV) irradiation, as measured according to the following Equation 1, and has a difference in yellowness index (ΔYI₂₋₀) of about 2.5 or less before and after UV irradiation, as measured according to the following Equation 2: [Equation 1] 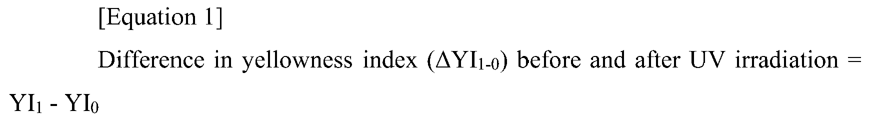 Difference in yellowness index (ΔYI1-0) before and after UV irradiation = YI1 - YI0 wherein YI₀ is a yellowness index of a 3.2 mm-thick specimen before UV irradiation, as measured according to ASTM D1925, and YI₁ is a yellowness index of the specimen after UV irradiation, as measured according to ASTM D1925 after irradiating the specimen with UV rays at 340 nm and 0.35 W/m² conditions at 65°C for 12,000 hours according to ASTM G155; [Equation 2] 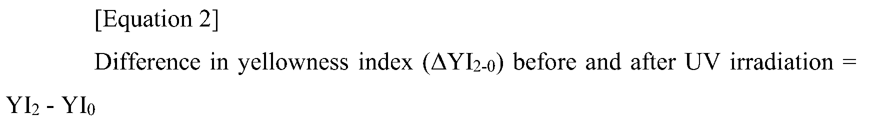 Difference in yellowness index (ΔYI2-0) before and after UV irradiation = YI2 - YI0 wherein YI₀ is a yellowness index of a 3.2 mm-thick specimen before UV irradiation, as measured according to ASTM D1925, and YI₂ is a predicted yellowness index value of the specimen after UV irradiation, as measured according to ASTM D1925 after irradiating the specimen with UV rays at 340 nm and 0.35 W/m² conditions at 65°C for 16,000 hours according to ASTM G155.

14. The eco-friendly ship hull of any one of claims 1 to 13, wherein the eco-friendly ship hull has a change in tensile strength of about 12% or less before and after UV irradiation, as measured according to the following Equation 3, and has a change in tensile strength of about 12% or less before and after UV irradiation, as measured according to the following Equation 4: [Equation 3] 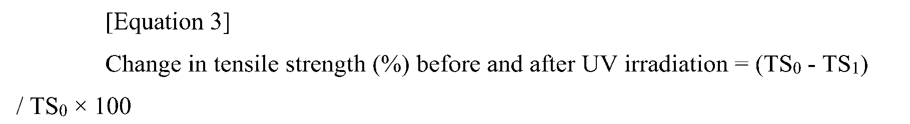 Change in tensile strength (%) before and after UV irradiation = (TS0 - TS1) / TS0 × 100 wherein TS₀ is a tensile strength of a 3.2 mm-thick specimen before UV irradiation, as measured according to ASTM D638, and TS₁ is a tensile strength of the specimen after UV irradiation, as measured according to ASTM D638 after irradiating the specimen with UV rays at 340 nm and 0.35 W/m² conditions at 65°C for 12,000 hours according to ASTM G155; [Equation 4] 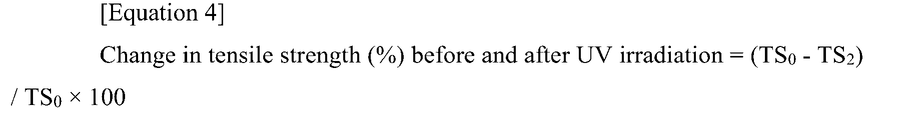 Change in tensile strength (%) before and after UV irradiation = (TS0 - TS2) / TS0 × 100 wherein TS₀ is a tensile strength of a 3.2 mm-thick specimen before UV irradiation, as measured according to ASTM D638, and TS₂ is a predicted tensile strength value of the specimen after UV irradiation, as measured according to ASTM D638 after irradiating the specimen with UV rays at 340 nm and 0.35 W/m² conditions at 65°C for 16,000 hours according to ASTM G155.

15. The eco-friendly ship hull of any one of claims 1 to 14, wherein the eco-friendly ship hull has an anti-slip surface shape having a cubic or grain shape.

16. The eco-friendly ship hull of any one of claims 1 to 15, wherein the eco-friendly ship hull has a glossy surface or a matte surface.

17. A method of manufacturing an eco-friendly ship hull, comprising:
mixing and melting high-density polyethylene, a carbon material, a light stabilizer, and an antioxidant and then extruding the resulting mixture to prepare pellets;
extrusion-molding the pellets into a sheet shape;
cutting the sheet into the shape of a ship hull part according to a nesting drawing; and
assembling (welding) the ship hull part.

18. The method of claim 17, wherein the pellets are prepared under extrusion molding temperature conditions of about 170 to about 240°C.

19. The method of claim 17 or 18, wherein the sheet has a thickness of about 5 mm or more and a width of about 500 mm or more.

20. The method of any one of claims 17 to 19, wherein the sheet has a single-layered structure or a multi-layered structure.
